# EUROPÄISCHE PATENTANMELDUNG

(11) **EP 1 057 493 A2**
(43) Veröffentlichungstag der Anmeldung: **06.12.2000**
(21) Anmeldenummer: 00111530.2
(22) Anmeldetag: 30.05.2000
(51) Int. Cl.: A61M 1/16

(54) **Kartuschenhalter für eine Dialysemaschine**

(30) Priorität: 02.06.1999 DE 19925297
(71) Anmelder: B. BRAUN MELSUNGEN AG, 34212 Melsungen (DE)
(72) Erfinder: Jacobi, Jürgen, 34212 Melsungen (DE); Mardorf, Robert, 34212 Melsungen (DE)
(74) Vertreter: Selting, Günther, Dipl.-Ing.

(57) **Zusammenfassung**

An einer Dialysemaschine ist ein Kartuschenhalter vorgesehen, an dem eine Filterkartusche (11) oder eine Dialysatorkartusche befestigt werden kann. Der Kartuschenhalter (25) weist ein die Kartusche (11) festhaltendes Halteorgan (35) und ein relativ zu dem Halteorgan radial zur eingesetzten Kartusche bewegbares Spannorgan (36) auf. Das Spannorgan kann von einer Öffnungsstellung in eine Schließstellung bewegt werden. Es weist zwei mit den seitlichen Anschlußstutzen (26, 27) der Kartusche zusammenwirkende Anschlußelemente (41, 42) auf, die beim Bewegen des Spannorgans (36) in die Schließstellung in abdichtenden Eingriff mit den beiden Anschlußstutzen (26, 27) der Kartusche kommen. Auf diese Weise wird durch einfaches Anstecken der Kartusche an den Kartuschenhalter eine Verbindung mit den beiden Anschlußstutzen der Kartusche hergestellt. Das einzelne Anschließen von Schläuchen an die Anschlußstutzen entfällt.

## Beschreibung

Die Erfindung betrifft einen Kartuschenhalter für eine Dialysemaschine, zur Befestigung und zum Anschluß einer Kartusche, die einen Filter oder einen Dialysator enthält.

Dialysemaschinen brauchen mindestens einen Filter zur Feinfilterung der Dialysierflüssigkeit, bevor diese dem Dialysator zugeführt wird. Dieser Filter ist üblicherweise ein in einer Kartusche enthaltener Hohlfaserfilter. Die Kartusche hat zwei an entgegengesetzten Enden befindliche axiale Anschlußstutzen, die an den Zulauf angeschlossen werden, und zwei von dem zylindrischen Kartuschenkörper seitlich abstehende Anschlußstutzen, die miteinander verbunden werden und den zum Dialysator führenden Filtratauslaß bilden. Der Dialysator besteht ebenfalls aus Hohlfasern, die in einer Kartusche angeordnet sind. Der Dialysatweg führt durch zwei seitlich vom Kartuschenkörper abstehende Anschlußstutzen hindurch und der Blutweg führt axial durch die Kartusche hindurch. Üblicherweise werden die Filterkartusche und die Dialysatorkartusche mit entsprechenden Schlauchleitungen der Dialysemaschine verbunden. Da Filter und Dialysator insgesamt acht Anschlüsse benötigen, kann es leicht vorkommen, daß Schlauchleitungen falsch angeschlossen werden, was verhängnisvolle Folgen haben kann. Außerdem ist ein Wechsel des Filters oder des Dialysators relativ aufwendig.

Der vorliegenden Erfindung liegt die Aufgabe zugrunde, einen Kartuschenhalter für eine Dialysemaschine zu schaffen, der das Verbinden einer Kartusche mit der Dialysemaschine erleichtert und Anschlußvertauschungen ausschließt.

Die Lösung dieser Aufgabe erfolgt erfindungsgemäß mit den im Patentanspruch 1 angegebenen Merkmalen.

Der erfindungsgemäße Kartuschenhalter weist einerseits ein Halteorgan auf, welches die Kartusche in definierter Lage festhält, und andererseits ein Spannorgan, welches zwei Anschlußelemente gleichzeitig mit den seitlichen Anschlußstutzen der Kartusche abdichtend verbindet, wenn es von einer Öffnungsstellung in die Schließstellung gebracht wird. Bei dem erfindungsgemäßen Kartuschenhalter ist es also nicht mehr erforderlich, Schlauchleitungen einzeln auf Anschlußstutzen der Kartusche aufzuschieben. Vielmehr wird die Kartusche durch das Halteorgan festgehalten und die Anschlußverbindungen werden durch das Spannorgan gleichzeitig hergestellt. Die Kartusche braucht also lediglich in den Kartuschenhalter eingesetzt zu werden und anschließend muß das Spannorgan betätigt werden. Ein Hantieren an den Anschlußstutzen der Kartusche ist nicht mehr erforderlich. Auch die Gefahr der Leitungsvertauschung besteht nicht, weil die Leitungen an den entsprechenden Anschlußelementen des Kartuschenhalters herstellermäßig fest angebracht sind und der Benutzer an diesen Leitungen überhaupt nicht mehr hantieren muß.

Die Erfindung eignet sich sowohl für Kartuschenhalter, die für Filterkartuschen bestimmt sind, als auch für Kartuschenhalter, die für Dialysatorkartuschen bestimmt sind. In beiden Fällen können allerdings die Kartuschengrößen voneinander abweichen, jedoch ist das Prinzip der Anschlußverbindungen in beiden Fällen im wesentlichen gleich. Ein Unterschied zwischen Filterkartuschen und Dialysatorkartuschen besteht darin, daß Filterkartuschen vier gleiche Anschlußstutzen aufweisen, während bei Dialysatorkartuschen die Anschlußstutzen für den Blutkreislauf anders ausgebildet sind als diejenigen für die Dialysierflüssigkeit. Erfindungsgemäß werden durch eine automatische Verbindungsvorrichtung die beiden seitlichen Anschlußstutzen der Kartusche durch einen Schließvorgang gleichzeitig mit den entsprechenden Anschlußelementen verbunden. Hierbei werden die Enden der Anschlußstutzen in Dichtringe der Anschlußelemente eingeschoben.

Das Halteorgan, mit dem die Kartusche während des Ankuppelns und im angekuppelten Zustand festgehalten wird, weist vorzugsweise Halteöffnungen zum Angreifen an den Anschlußstutzen der Kartusche auf. Diese Halteöffnungen sind als Schlüssellochöffnungen ausgebildet und sie sind so gestaltet, daß ihre Ränder in Nuten der Anschlußstutzen der Kartusche eindringen, so daß die Kartusche gegen Herausziehen gesichert ist. Zum Entfernen der Kartusche muß diese in den Schlüssellochöffnungen verschoben werden, bis die Anschlußstutzen in den breiteren Kopfbereich der Schlüssellochöffnungen gelangen, aus denen sie herausgezogen werden können.

Gemäß einer bevorzugten Weiterbildung der Erfindung ist das Spannorgan durch einen an dem Halteorgan angelenkten Schwenkbügel bewegbar, welcher in seiner Schließstellung eine Verriegelung der eingesetzten Kartusche bewirkt. Der Schwenkbügel bildet eine Sicherung gegen unbeabsichtigtes Herausziehen der Kartusche aus dem Kartuschenhalter sowie auch eine Sicherung gegen jegliche Art von Bewegung der Kartusche.

Der Kartuschenhalter kann in der Weise weitergebildet sein, daß er nicht nur die Verbindung zu den seitlichen Anschlußstutzen der Kartusche herstellt, sondern auch die Verbindung zu den axialen Anschlußstutzen. Hierbei ist zweckmäßigerweise ein Anschlußelement für einen axialen Anschlußstutzen in den Schwenkbügel integriert. Beim Schließen des Schwenkbügels kommt das axiale Anschlußelement in abdichtenden Eingriff mit dem entsprechenden Anschlußstutzen der Kartusche. Am gegenüberliegenden Ende der Kartusche ist eine Stützvorrichtung vorgesehen, die ein weiteres Anschlußelement für einen axialen Anschlußstutzen der Kartusche aufweist.

Im folgenden werden unter Bezugnahme auf die Zeichnungen Ausführungsbeispiele der Erfindung näher erläutert.

Es zeigen:
- Fig. 1: ein schematisches Fließschaubild eines Filters und eines Dialysators bei einer Dialysemaschine,
- Fig. 2: einen Vertikalschnitt durch einen Kartuschenhalter mit eingesetzter Kartusche im geöffneten Zustand,
- Fig. 3: eine Frontansicht von Fig. 2 aus Richtung des Pfeiles III,
- Fig. 4: in gleicher Darstellung wie Fig. 2 den Kartuschenhalter im Betriebszustand, in dem der Schwenkbügel geschlossen ist,
- Fig. 5: einen Schnitt entlang der Linie V-V von Fig. 4,
- Fig. 6: einen Kartuschenhalter für eine Dialysatorkartusche in Seitenansicht, teilweise geschnitten,
- Fig. 7: eine Frontansicht des Kartuschenhalters von Fig. 6,
- Fig. 8: in gleicher Darstellung wie Fig. 7 den Kartuschenhalter im verschwenkten Zustand zur Prüfung auf Blasen und
- Fig. 9: den Kartuschenhalter der Fign. 6-8 im verlängerten Zustand und mit einem die Anschlußelemente verbindenden Kurzschlußbügel.

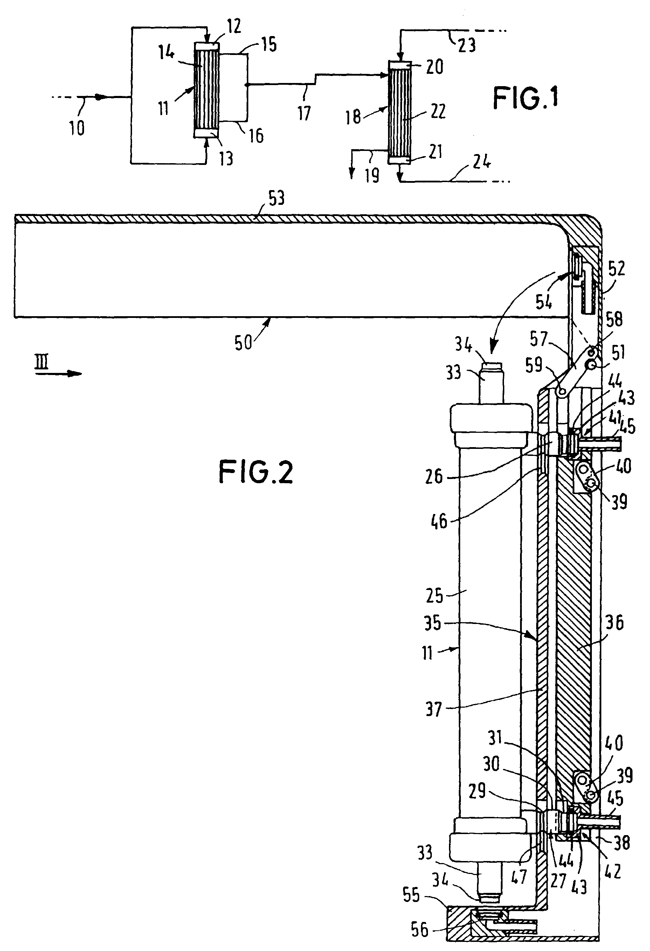

In Fig. 1 ist schematisch ein Teil des Dialysatweges und des Blutweges einer Dialysemaschine dargestellt. Die Dialysierflüssigkeit wird durch eine Leitung 10 den beiden stirnseitigen Enden einer Filterkartusche 11 zugeführt. Die Filterkartusche enthält in ihrem einen Ende eine Kammer 12 und am anderen Ende eine Kammer 13. Zwischen diesen Kammern 12, 13 erstreckt sich ein Hohlfaserbündel 14, dessen Hohlfasern von der Dialysierflüssigkeit durchströmt werden. Die Hohlfaserwände sind für die Dialysierflüssigkeit durchlässig, jedoch lassen sie darin enthaltene Partikel nicht durch. Die Kartusche 11 weist zwei seitliche Auslässe 15, 16 auf, die untereinander verbunden und durch eine Leitung 17 mit einem seitlichen Anschluß der Dialysatorkartusche 18 verbunden sind. Die Dialysierflüssigkeit strömt an den Hohlfasern entlang und wird durch einen seitlichen Anschluß 19 abgeleitet. Die Dialysatorkartusche 18 weist ebenfalls an den Stirnseiten jeweils eine Kammer 20 bzw. 21 auf. Zwischen diesen Kammern verlaufen Hohlfasern 22. Das Blut wird durch die Zulaufleitung 23 vom Patienten zugeführt und verläßt den Dialysator durch die Auslaßleitung 24.

In den Fign. 2 bis 5 ist ein an einer (nicht dargestellten) Dialysemaschine angebrachter Kartuschenhalter für eine Filterkartusche 11 gezeigt. Die Filterkartusche 11 weist einen rohrförmigen Kartuschenkörper 25 auf, der mit zwei seitlich abgehenden Anschlußstutzen 26, 27 versehen ist. Diese Anschlußstutzen sind untereinander gleich. Sie weisen in der Nähe des Kartuschenkörpers eine umlaufende Nut 29 auf, an die sich nach außen hin eine Verdickung 30 anschließt. Das äußere Ende des Anschlußstutzens bildet eine zylindrische Dichtfläche 31 von geringerem Durchmesser als die Verdickung 30.

Die Filterkartusche 11 hat ferner zwei axiale Anschlußstutzen 32, 33 mit jeweils einer am Ende vorgesehenen zylindrischen Dichtfläche 34 von verringertem Durchmesser.

Der Kartuschenhalter weist ein Halteorgan 35 auf, das die Trag- und Haltevorrichtung bildet und an dem ein Spannorgan 36 bewegbar befestigt ist. Das Halteorgan 35 ist generell U-förmig und besteht aus einer Frontplatte 37 und seitlichen Schenkeln 38. In seinem Innern ist das aus einer Platte bestehende Spannorgan 36 angeordnet. Zwischen den Schenkeln 38 erstrecken sich Achsen 39, an denen jeweils ein Lenker 40 gelagert ist. Das andere Ende des Lenkers 40 ist gelenkig mit dem Spannorgan 36 verbunden. Auf diese Weise bilden das Halteorgan 35, das Spannorgan 36 und die Lenker 40 ein Parallelogrammgestänge. Das Spannorgan 36 kann unter Beibehaltung seiner Parallelausrichtung in bezug auf das Halteorgan 35 verschwenkt werden, wobei es an die Frontplatte 37 angenähert oder von dieser weg bewegt wird.

Das Spannorgan 36 trägt zwei Anschlußelemente 41, 42. Jedes Anschlußelement besteht aus einer Fassung 43 mit einem eingesetzten Dichtring 44, in den die zylindrische Dichtfläche 31 des Anschlußstutzens 26 bzw. 27 abdichtend eingeführt werden kann. An ihrem rückwärtigen Ende ist die Fassung 43 mit einem nach hinten ragenden Schlauchanschluß 45 versehen.

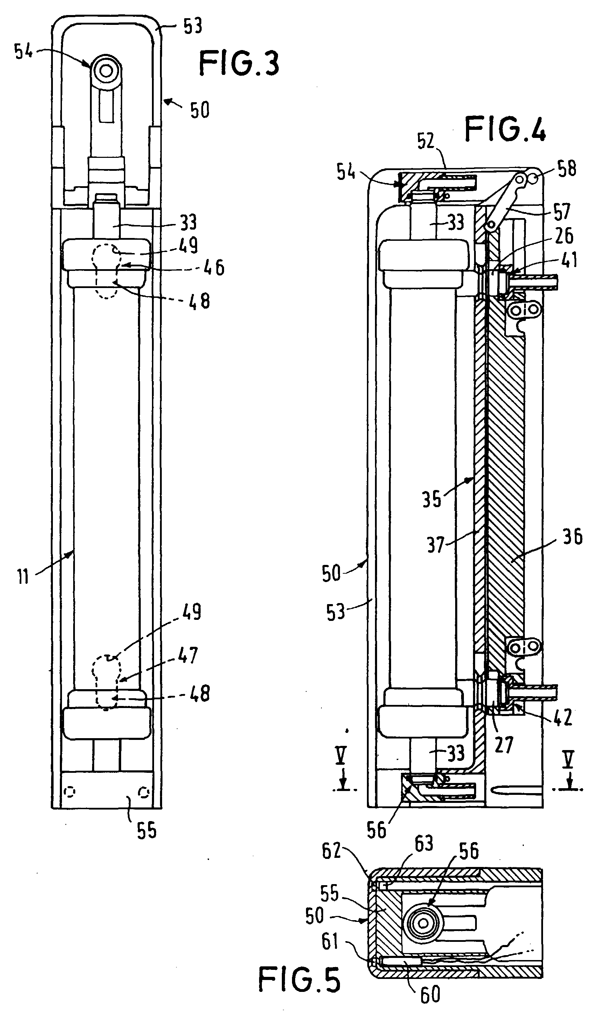

Die Frontplatte 37 des Halteorgans 35 ist mit Halteöffnungen 46, 47 versehen, die schlüssellochförmig ausgebildet sind. Dies bedeutet, daß jede Halteöffnung einen relativ schmalen vertikalen Schlitz 48 und eine breitere kreisrunde Kopföffnung 49 aufweist (Fig. 3). Die Kopföffnung 49 dient zum Einschieben des seitlichen Anschlußstutzens 26 bzw. 27 und der Schlitz 48 hat eine solche Breite, daß seine Schlitzränder in die Nut 29 des Anschlußstutzens eingreifen und den Anschlußstutzen somit in Achsrichtung fixieren. Die Kartusche wird so eingesetzt, daß die beiden Anschlußstutzen 26, 27 durch die Kopföffnungen 49 hindurchgesteckt werden, und anschließend wird die Kartusche heruntergezogen, um in den Schlitzen 48 festgelegt zu werden. Nach dieser Fixierung wird das Spannorgan 36 in Richtung auf die Frontplatte 37 bewegt (Fig. 4), so daß die Fassungen 43 mit den Dichtringen 44 über die Dichtflächen 31 der Anschlußstutzen geschoben werden. Dadurch werden die Anschlußstutzen in jeder Richtung fixiert.

Die horizontale Parallelbewegung des Spannorgans 36 wird durch einen Schwenkbügel 50 bewirkt, der um eine horizontale Achse 51 am oberen Ende des Halteorgans 35 schwenkbar ist. Der Schwenkbügel 50 ist L-förmig und er weist einen an die Achse 51 angrenzenden kurzen Dachschenkel 52 und einen davon rechtwinklig abstehenden Längsschenkel 53 von U-förmigem Querschnitt auf. Im heruntergeschwenkten Zustand (Fig. 4) umschließt der Längsschenkel 53 die Kartusche 11 von drei Richtungen her. In der vierten Richtung ist die Kartusche von dem Halteorgan umschlossen. Der Schwenkbügel 50 erstreckt sich über die gesamte Kartuschenlänge. Der Dachschenkel 52 des Schwenkbügels 50 enthält ein Anschlußelement 54, das ähnlich ausgebildet ist wie die Anschlußelemente 41 und 42. Beim Schließen des Schwenkbügels 50 wird das Anschlußelement 54 um 90° verschwenkt und mit vertikaler Achse auf das Ende des Anschlußstutzens 33 der Kartusche aufgeschoben. Der Schwenkbügel 50 ist zugleich als Wärmeschutzhaube ausgebildet, um Temperaturverluste, speziell beim Desinfizieren, zu reduzieren.

Das Halteorgan 35 ist am unteren Ende mit einer vorstehenden Stützvorrichtung 55 versehen, die ein weiteres Anschlußelement enthält, das für den Anschlußstutzen 33 der Kartusche vorgesehen ist. Das Anschlußelement 56 ist mit vertikaler Achse ausgerichtet. Bei geöffnetem Schwenkbügel 50 befindet sich der untere Anschlußstutzen 33 mit axialem Abstand oberhalb des Anschlußelements 56.

Die Steuerung des Spannorgans 36 erfolgt durch einen Lenker 57, der mit einem Gelenk 58 mit dem Schwenkbügel 50 und mit einem Gelenk 59 mit dem Spannorgan 36 verbunden ist. Beim Schließen des Schwenkbügels 50 wird über den Lenker 57 das Spannorgan 36 heruntergedrückt. Infolge der schrägstehenden Lenker 40 führt das Spannorgan dabei eine Vorwärtsbewegung in Richtung auf die Frontplatte 37 durch. Dabei werden die Fassungen 41, 42 auf die Enden der Anschlußstutzen 25, 27 aufgeschoben. Ferner werden die Anschlußstutzen in den jeweiligen Halteöffnungen 46, 47 im Bereich des Schlitzes 48 herunterbewegt.

In Fig. 5 ist der U-förmige Schwenkbügel 50 im geschlossenen Zustand dargestellt, in dem sein unteres Ende die Stützvorrichtung 55 umschließt. In der Stützvorrichtung 55 ist ein Magnetsensor 60 vorgesehen, der auf einen Magneten 61 des Schwenkbügels 50 anspricht und dadurch den Schließzustand des Schwenkbügels detektiert. Der Sensor 60 läßt einen Betrieb der Dialysemaschine nur bei geschlossenem Schließbügel zu. Im Schwenkbügel 50 und in der Stützvorrichtung 55 befinden sich ferner Magnete 62, 63, die den Schwenkbügel in die Schließstellung hineinziehen.

Zum Einsetzen der Kartusche 11 wird diese mit den Anschlußstutzen 26, 27 durch die Kopföffnungen 49 der schlüssellochförmigen Halteöffnungen 45, 47 gesteckt, während das Spannorgan 36 sich bei geöffnetem Schwenkbügel 50 in der Rückzugsstellung befindet. Dann wird die Kartusche 11 heruntergedrückt, so daß die Anschlußstutzen 26, 27 in den Bereich der Schlitze 48 gelangen und sich dann in axialer Ausrichtung zu dem jeweiligen Anschlußelement 41, 42 befinden. In diesem Zustand wird der Schwenkbügel 50 von der Öffnungsstellung in die Schließstellung geschwenkt. Dabei wird auch das Spannorgan 36 in die Schließstellung vorgeschoben. Die Anschlußelemente 41, 42 werden dabei auf die Anschlußstutzen 26, 27 aufgeschoben und die Anschlußstutzen werden in der jeweiligen Halteöffnung 46, 47 noch weiter im Schlitz 48 herunterbewegt. Bei dieser Abwärtsbewegung wird der untere axiale Anschlußstutzen 33 in das entsprechende Anschlußelement 56 der Stützvorrichtung 55 eingeführt. Am Ende der Schwenkbewegung des Schwenkbügels wird das obere Anschlußelement 54 abdichtend auf den oberen Anschlußstutzen 33 der Kartusche aufgeschoben. Nunmehr sind sämtliche Anschlußstutzen abdichtend mit den jeweiligen Anschlußelementen verbunden und die Kartusche kann in Betrieb genommen werden.

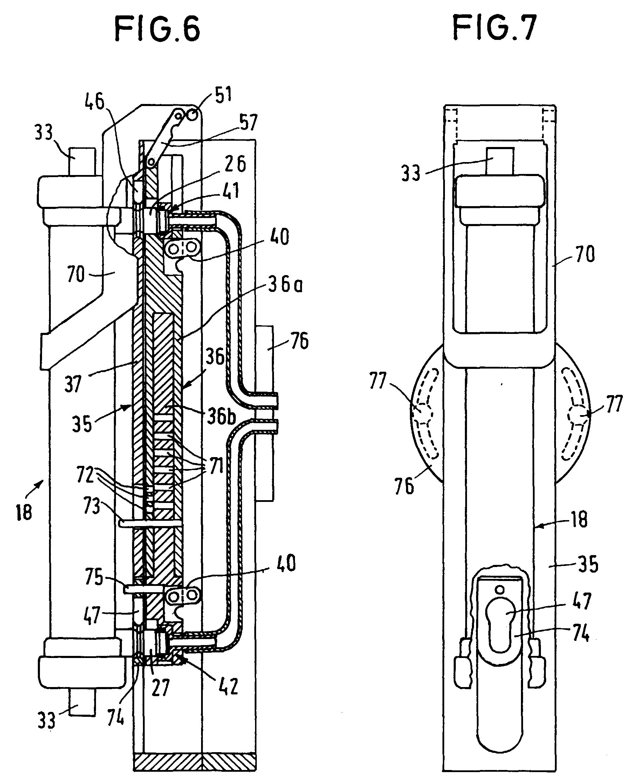

In den Fign. 6-9 ist ein Kartuschenhalter für eine Dialysatorkartusche 18 dargestellt. Diejenigen Teile, die denjenigen des ersten Ausführungsbeispiels entsprechen, sind mit den gleichen Bezugszeichen versehen. Die nachfolgende Beschreibung beschränkt sich auf die Darlegung der Unterschiede.

Im Gegensatz zu der Filterkartusche werden bei der Dialysatorkartusche von dem Kartuschenhalter nur die beiden seitlichen Anschlußstutzen 26 und 27 angeschlossen, während die axialen Anschlußstutzen 33 manuell an die entsprechenden Schlauchleitungen angeschlossen werden. Demgemäß ist der Schwenkbügel 70 bei dem vorliegenden Ausführungsbeispiel kleiner ausgebildet, so daß er zwar die Kartusche 18 in Draufsicht von drei Seiten umgibt, sich aber nur über einen Teil der Länge der Kartusche erstreckt. Auch hier steuert der Schwenkbügel 70 die Bewegung des Spannorgans 36, das über Lenker 40 mit dem Halteorgan 35 verbunden ist.

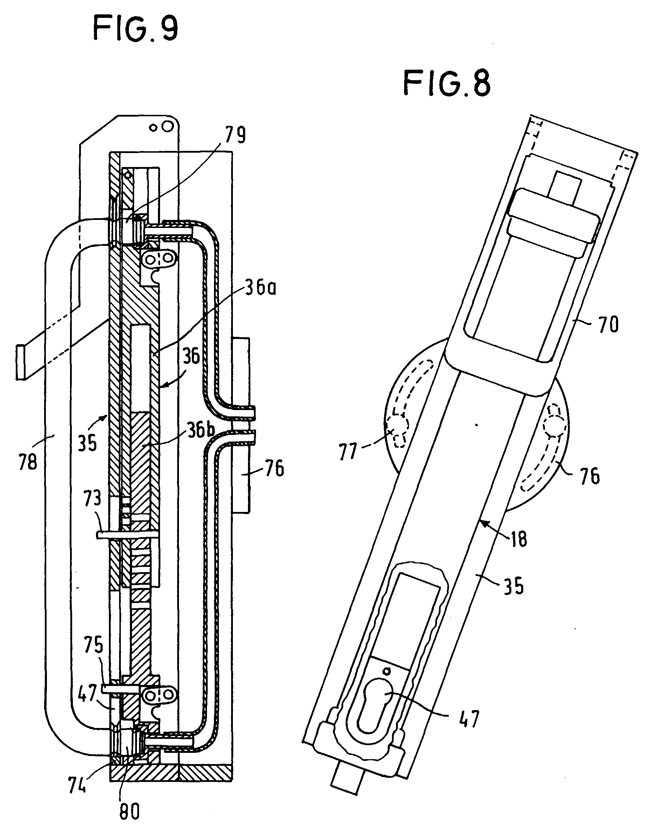

Das Spannorgan 36 ist bei dem vorliegenden Ausführungsbeispiel längenverstellbar. Zu diesem Zweck ist in einen Körper 36a des Spannorgans ein Schieber 36b eingeschoben. Der Schieber enthält mehrere Löcher 71, die jeweils mit einem Gegenloch 72 in dem Körper 36a ausgerichtet und durch Hindurchstecken eines Steckstiftes 73 fixiert werden können. An dem unteren Ende des Schiebers 36b befindet sich das Anschlußelement 42 und an diesem Ende greift auch der untere Lenker 40 an. In Fig. 6 ist das Spannorgan im eingefahrenen Zustand dargestellt und in Fig. 9 in einem herausgefahrenen Zustand. An dem unteren Ende des Schiebers 36b ist, dem Anschlußelement 42 vorgelagert, ein Mitnehmer 74 angebracht, an dem sich die untere Halteöffnung 47 befindet. Dieser Mitnehmer ist über einen Mitnehmerstift 75 mit dem Unterteil des Schiebers 36b gekoppelt, so daß die Halteöffnung 47 sich immer vor dem unteren Anschlußelement 42 befindet.

An der Rückseite des Halteorgans 35 ist eine Schwenkvorrichtung 76 angebracht, die mit Schrauben 77 am Gehäuse der Dialysemaschine befestigt wird. Durch Lösen der Schrauben 77 kann der gesamte Kartuschenhalter um eine horizontale Querachse herumgeschwenkt werden. Dies dient dazu, durch Sichtkontrolle in der durchsichtigen Kartusche 18 Luftblasen erkennen zu können. Im Betriebszustand wird der Kartuschenhalter in die senkrechte Position gebracht, die in den Fign. 6 und 7 dargestellt ist.

In Fig. 9 ist der Kartuschenhalter im ausgezogenen Zustand dargestellt. Hierbei ist in das Halteorgan keine Kartusche eingesetzt, sondern ein Kurzschlußbügel 78 mit an den Enden vorgesehenen Anschlußstutzen 79 und 80, die in gleicher Weise ausgebildet sind wie die Anschlußstutzen einer Kartusche. Mit dem Kurzschlußbügel kann eine Spülung des Leitungssystems erfolgen.

## Patentansprüche

1. Kartuschenhalter für eine Dialysemaschine zur Befestigung und zum Anschluß einer Kartusche (11;18), die einen rohrförmigen Kartuschenkörper (25) und zwei seitlich von dem Kartuschenkörper abstehende Anschlußstutzen (26, 27) aufweist, mit einem die Kartusche festhaltenden Halteorgan (35) und einem relativ zu dem Halteorgan radial zur eingesetzten Kartusche bewegbaren Spannorgan (36), welches von einer Öffnungsstellung in eine Schließstellung bewegbar ist und zwei mit den seitlichen Anschlußstutzen (26, 27) der Kartusche zusammenwirkende Anschlußelemente (41, 42) aufweist, die beim Bewegen des Spannorgans (36) in die Schließstellung in abdichtenden Eingriff mit den beiden Anschlußstutzen (26, 27) kommen.

2. Kartuschenhalter nach Anspruch 1, dadurch gekennzeichnet, daß das Spannorgan (36) aus einer durch Lenker (40) mit dem Halteorgan (35) parallelverschiebbar verbundenen starren Struktur besteht.

3. Kartuschenhalter nach Anspruch 1 oder 2, dadurch gekennzeichnet, daß das Halteorgan (35) Halteöffnungen (46, 47) zum Angreifen an den Anschlußstutzen (26, 27) aufweist.

4. Kartuschenhalter nach Anspruch 3, dadurch gekennzeichnet, daß die Halteöffnungen (46, 27) als Schlüssellochöffnungen ausgebildet sind und die Anschlußstutzen (26, 27) Nuten (29) aufweisen, die passend in dem Schlitz (48) der Schlüssellochöffnungen aufgenommen werden.

5. Kartuschenhalter nach einem der Ansprüche 1-4, dadurch gekennzeichnet, daß das Spannorgan (36) durch einen an dem Halteorgan (35) angelenkten Schwenkbügel (50; 70) bewegbar ist, welcher in seiner Schließstellung die Verriegelung der eingesetzten Kartusche bewirkt.

6. Kartuschenhalter nach Anspruch 5, dadurch gekennzeichnet, daß der Schwenkbügel (50; 70) im Schließzustand die eingesetzte Kartusche (11;18) von drei Seiten umfängt.

7. Kartuschenhalter nach Anspruch 5 oder 6, dadurch gekennzeichnet, daß der Schwenkbügel (50) ein axiales Anschlußelement (54) enthält, das beim Bewegen des Schwenkbügels in die Schließstellung in abdichtenden Eingriff mit einem axialen Anschlußstutzen (33) der Kartusche (11) kommt.

8. Kartuschenhalter nach Anspruch 7, dadurch gekennzeichnet, daß das Halteorgan (35) an dem dem Schwenkbügel (50) gegenüberliegenden Ende eine Stützvorrichtung (55) aufweist, die ein weiteres Anschlußelement (56) für einen axialen Anschlußstutzen (33) der Kartusche (11) aufweist.

9. Kartuschenhalter nach einem der Ansprüche 1-8, dadurch gekennzeichnet, daß das Spannorgan (36) zur Anpassung an Kartuschen unterschiedlicher Größen in seiner Länge verstellbar ist.

10. Kartuschenhalter nach einem der Ansprüche 1-9, dadurch gekennzeichnet, daß das Halteorgan (35) mit einer Schwenkvorrichtung (76) an der Dialysemaschine befestigt ist, welche das Verschwenken der eingesetzen Kartusche (18) um eine horizontale Querachse ermöglicht.

11. Kartuschenhalter nach Anspruch 5, dadurch gekennzeichnet, daß ein Sensor (60) vorgesehen ist, der die Schließstellung des Schwenkbügels (50) erkennt und einen Betrieb der Dialysemaschine nur bei geschlossenem Schließbügel zuläßt.
